(19) Europäisches Patentamt
European Patent Office
Office européen des brevets

(11) Publication number: **0 096 463**
**B1**

(12) # EUROPEAN PATENT SPECIFICATION

(45) Date of publication of patent specification: **03.12.86**

(21) Application number: **83302514.1**

(22) Date of filing: **04.05.83**

(51) Int. Cl.⁴: **C 07 K 3/08,** C 07 K 13/00, A 61 K 39/395, G 01 N 33/542, G 01 N 33/531

(54) **Immunoglobulin half-molecules and process for producing hybrid antibodies.**

(30) Priority: **05.05.82 US 374971**
**05.05.82 US 374970**

(43) Date of publication of application:
**21.12.83 Bulletin 83/51**

(45) Publication of the grant of the patent:
**03.12.86 Bulletin 86/49**

(84) Designated Contracting States:
**BE DE FR GB IT LU NL**

(56) References cited:
**EP-A-0 035 265**
**DE-A-2 508 132**
**DE-A-2 805 961**
**DE-A-3 134 361**

**Chemical Abstracts vol. 63, No. 9, 25 october 1965, Columbus, Ohio, USA R. HONG et al. "Relative labilities of the two types of interchain disulfide bond of rabbit gammaG-immunoglobulin" column 11903c-e**
**Chemical Abstracts vol. 86, no. 25, 20 June 1977, Columbus, Ohio, USA D.W. SEARS et al.**

(73) Proprietor: **E.I. DU PONT DE NEMOURS AND COMPANY**
**1007 Market Street**
**Wilmington Delaware 19898 (US)**

(72) Inventor: **Auditore-Hargreaves, Karen**
**1400 Forrest Drive**
**Wilmington Delaware 19810 (US)**
Inventor: **Miesowicz, Frederick Michael**
**11 Whitekirk Drive**
**Wilmington Delaware, 19808 (US)**

(74) Representative: **Myerscough, Philip Boyd et al**
**J.A.Kemp & Co. 14, South Square Gray's Inn**
**London, WC1R 5EU (GB)**

(56) References cited:
**"Acquisition of the covalent quaternary structure of an immunoglobulin G molecule. Reoxidative assembly in vitro" page 414, column 2, abstract no. 187466x**
**Chemical Abstracts vol. 93, no. 7, 18 August 1980, Columbus, Ohio, USA D.S. PEABODY et al. "Obligatory hybridization of heterologous immunoglobulin light chains into covalently linked dimers" page 702, column 1, abstract no. 68341n**
**Microbiology III Edition Harper Into Ed. B Davis R. Dulbeno et al. p. 333-334**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 096 463 B1

## Description

This invention relates to a process for preparing immunoglobulin half-molecules and more particularly to the selective rejoining of dissimilar half-molecules of IgG antibodies to form covalent hybrid antibodies. These antibodies can be utilized for the measurement of large molecular weight antigens and haptens in biological fluids.

Hybrid antibodies of the IgG class are heterobifunctional antibodies having two binding sites for antigens, each site having been derived from a different immunoglobulin. Usually, each of the two binding sites recognizes a different antigen, but it is possible for both binding sites to bind the same antigen and to differ with respect to other properties, for example, idiotype.

The synthesis of hybrid antibodies has been achieved in low yields by means of reductive dissociation of each of two intact immunoglobulin molecules ($H_2L_2$), having different antigenbinding specificities, into half-molecules ($H_1L_1$), where H represents the heavy chain and L, the light chain of immunoglobulin, followed by reoxidation of a mixture of these half-molecules, see, for example, Hong, et al., J. Biol. Chem., Volume 240, 3883 (1965). This purely random reoxidation has the disadvantage of yielding a theoretical maximum of 50% of the desired hybrid product.

Luedtke, et al., Biochemistry, Volume 19, 1182 (1980), describe the preparation of a hybrid antibody, in a mixture with the parent immunoglobulin molecules, resulting from the random reassociation of two immunoglobulin half-molecules. After removal of one parent immunoglobulin, the resulting immunoglobulin population contained approximately 46% of the desired hybrid antibody. No further purification to obtain the desired hybrid antibody was carried out.

Heterologous recombination of immunoglobulin light chains to form dimers has been carried out by means of a nucleophilic displacement reaction; see Peabody, et al., Biochemistry, Volume 19, 2827 (1980). Immunoglobulin light chains are synthesized in abnormally high concentrations in certain diseased patients, e.g., multiple myeloma patients, and are excreted into the urine. Protein purification carried out on such samples led to light chain dimers. Splitting of light chain dimers from one source by sulfitolysis of the interchain disulfide bond afforded a thiosulfate derivative. Light chain dimers from a second source were split by reduction of the interchain disulfide bond to yield the corresponding sulfhydryl compound. Admixture of these two monomeric species, in turn, led to the formation of heterologous light chain, disulfide-linked dimers by a nucleophilic displacement reaction. Yields of 80 to 100% were reported. These light chain dimers are covalently bonded by a single disulfide bond and are not immunologically active. Native immunoglobulin molecules, on the other hand, have more than one disulfide bond and the specific cleavage of the inter-heavy chain disulfide bond(s) to form the desired half-molecule is difficult.

The reduction of the inter-heavy chain disulfide bond(s) of intact IgG molecules to form half-molecules by chemical means is difficult without concurrently causing some reduction of heavy-light chain disulfide bond(s) as well; see Sears, et al., Biochemistry, Volume 16, 2031 (1977). Limited proteolysis of IgG to yield $F(ab')_2$ fragments has been thought to be necessary to selectively enhance the susceptibility of the inter-heavy chain bond(s) to chemical attack; see Bobrzecka, et al., Immunology Letters, Volume 2, 15 (1980). Such limited proteolysis yields a mixture of products requiring subsequent chromatographic purification.

Rivat, et al., Eur. J. Immunol., Volume 3, 537 (1973), describe a procedure by which the inter-heavy chain disulfide bonds of immunoglobulins are reduced by electrochemical means to yield half-molecules with free sulfhydryl groups. These half-molecules, however, could not be isolated because they were subject to spontaneous reoxidation and homologous reassociation. An alkylation process was used for the analysis of these half-molecules but even this reaction had to be carried out in a nitrogen atmosphere.

Competitive binding immunoassays are widely used to determine qualitatively or quantitatively the presence of a ligand in a liquid medium. One type of such assays, referred to as homogeneous immunoassays, utilizes a specific ligand receptor which interacts with a ligand-indicator conjugate to modify the indicator in a measureable way. The indicator is usually an enzyme.

United States Patent 3,817,837, issued June 18, 1974 to Rubenstein, et al., discloses a homogeneous competitive binding immunoassay in which the activity of the indicator label in a label-ligand conjugate is modulated by the binding of the antibody to the ligand.

United States Patent 3,935,074, issued January 27, 1976 to Rubenstain, et al., discloses a method of utilizing a reagent having two epitopes, one common to the ligand of interest and one foreign to the ligand (the indicator label). Simultaneous binding of two antibodies to the epitopes is sterically inhibited. Measurement of the bound or unbound antibody to the indicator label epitope is used to provide a measure of the quantity of ligand in the unknown sample.

Hammerling, et al., J. Exp. Med., Volume 128, 1461 (1968), discloses the electron microscopic visualization of cell surfaces using antibodies consisting of two Fab' fragments linked by a disulfide bond. One Fab' is specific for a cell surface antigen and the other is specific for a marker which is visible by electron microscopy. This assay technique is not generally adaptable to soluble antigens and requires sophisticated instrumentation.

European patent application No. 034,050, published August 19, 1981 discloses a homogeneous immunoassay for antigens and haptens utilising a mixed binding reagent containing an

antigen-binding site and a label-binding site, the two sites being spaced apart so that a single molecule of an antigen-label conjugate cannot bind to both sites. The label is one whose activity is changed upon binding to the label-binding site of the mixed binding reagent. Measurement of the activity of the label is indicative of the amount of antigen in a liquid sample. The mixed binding reagent consists of two different intact immunoglobulin molecules being linked together to form a unitary reagent complex.

There is a need for an efficient method for the production of IgG half-molecules from intact IgG. There is also a need for a practical method for the synthesis of covalent hybrid antibodies from such half-molecules. There are discernible advantages in using covalent hybrid antibodies in homogeneous immunoassays for soluble antigens.

The half-molecules of this invention are substantially pure immunoglobulin heavy chain-light chain half-molecules having the structure R—S—$SO_3$—, where R is $H_1L_1$. They are prepared by selectively cleaving an immunoglobulin molecule into its heavy chain-light chain half-molecules by sulfitolysis of the inter-heavy chain disulfide linkage with sodium sulfite in the presence of 5,5'-dithiobis(2-nitrobenzoic acid).

The antibodies of this invention are substantially pure covalent hybrid antibodies consisting essentially of two different heavy chain-light chain half-molecules, wherein the first of the half-molecules provides a binding site for a first antigen and the second of the half-molecules provides a chemically different binding site for the first of a second antigen, and wherein the half-molecules are bonded to each other through disulfide linkage. These antibodies are prepared through the steps of:

(A) selectively cleaving a first immunoglobulin molecule which is an antibody to a first antigen into its heavy chain-light chain half-molecules by sulfitolysis of the inter-heavy chain disulfide bond with sodium sulfite in the presence of 5,5'-dithiobis-nitrobenzoic acid) to produce S-sulfonated half-molecule;

(B) selectively cleaving a second immunoglobulin molecule which is an antibody to the first or a second antigen into its heavy chain-light chain half-molecules by reduction of the inter-heavy chain disulfide bond to produce reduced half-molecules; and

(C) combining the S-sulfonated half-molecules from step (A) with the reduced half-molecules from step (B).

The homogeneous immunoassay of this invention for the measurement of various analytes comprises the steps of incubating sequentially or simultaneously:

(A) a substantially pure covalent hybrid antibody consisting essentially of two different heavy chain-light chain half-molecules, wherein the first of said half-molecules provides a binding site for a first antigen and the second of said half-molecules provides a chemically different binding site for the first or a second antigen; wherein said half-molecules are bonded to each other through disulfide linkage;

(B) biological sample containing analyte;

(C) an indicator; and

(D) reagents for signal-generating reaction.

The process of this invention for preparing covalent hybrid antibodies comprises several different methods for the selective cleavage of the inter-heavy chain disulfide bond(s) of intact IgG to yield heavy chain-light chain half-molecules ($H_1L_1$), as well as the ordered recombination of dissimilar half-molecules thus obtained to yield covalent hybrid antibodies ($H_1^aL_1^aH_1^bL_1^b$). The dissimilar halves of the hybrid antibodies are held together by disulfide linkage. This process results in high yields of the desired product often without the need for purification. The two half-molecules provide two chemically different binding sites for either two different antigens or for the same antigen but at differing locations on the antigen.

The process of this invention is a multistep procedure. The first step in the process of this invention is the selective cleavage of the inter-heavy chain disulfide bond(s) of two different intact IgG molecules. The use of monoclonal antibodies is also contemplated. Half-molecules formed from the parent IgG molecules are then combined in such a manner as to favor disulfide-bond formation between dissimilar half-molecules.

Selective cleavage of the inter-H chain bond and the production of IgG half-molecules can be achieved in several ways. In one method, intact IgG is sulfitolyzed with sodium sulfite in the presence of 5,5'-dithiobis(2-nitrobenzoic acid), preferably in a buffered medium at room temperature under nitrogen, to yield S-sulfonated half-molecules of IgG. One of the two half-molecules of IgG which can be used in the hybrid-forming reaction is S-sulfonated in this manner.

In a second method, S-sulfonated half-molecules of IgG formed as described above are reacted with a thiol-containing reagent such as dithiothreitol, β-mercaptoethanol and β-mercaptoethylamine, preferably in a buffered medium containing strontium chloride, under nitrogen. Separation of the protein products of this reaction from the thiol reagent can be subsequently effected by gel filtration or dialysis. The protein products of this reaction are reduced half-molecules of IgG bearing free sulfhydryl group(s).

In a third method, intact IgG is first allowed to bind to a receptor, such as Protein A or antigen, covalently bound to a suitable support (e.g., agarose beads). A thiol-containing reagent such as dithiothreitol or β-mercaptoethanol is then reacted with the bound IgG (at room temperature, in a buffered medium, under nitrogen). The resultant bound IgG half-molecules having free sulfhydryl groups can then be dissociated from the support using a high-salt, low-pH buffer, and dialyzed against the same buffer to remove the thiol reagent.

The second of the two half-molecules of IgG which can be used in the hybrid-forming reaction is a free sulfhydryl-containing half-molecule prepared according to one of the methods described above.

The ordered hybridization of IgG molecules to provide hybrid antibodies having different antigen-binding specificites can be accomplished by different procedures. In one procedure, S-sulfonated half-molecules are derived from a first IgG by sulfitolysis as described above. The protein product (immunoglobulin half-molecules) can be separated from the other products of the sulfitolysis reaction by dialysis against a suitably buffered medium through which nitrogen is bubbled at room temperature. Sulfhydryl-containing half-molecules are derived from a second IgG by one of the methods described above. Removal of the thiol reagent, where one is present, can be accomplished without reoxidation of the protein either by gel filtration or by dialysis in a buffered medium of pH 5, containing, for example, ethylene diamine tetraacetic acid, which has been thoroughly degassed and through which nitrogen is bubbled. The two different populations of half-molecules generated by these methods are then combined in equimolar amounts and dialyzed under anaerobic conditions in a suitably buffered medium (containing strontium chloride or another alkali earth metal salt). A nucleophilic displacement reaction, shown below, affords the desired hybrid antibody product in which the dissimilar halves are joined together by disulfide linkage.

$$R{-}S{-}SO_3^- + R'{-}SH \rightarrow R{-}S{-}S{-}R' + HSO_3^-$$

where

$$R \text{ is } H_1^aL_1^a$$

and

$$R' \text{ is } H_1^bL_1^b$$

In another procedure of hybridization, S-sulfonated half-molecules are derived from a first IgG by sulfitolysis, as described above. Sulfhydryl-containing half-molecules are derived from a second IgG as described above in the third method for selective cleavage of the inter-H chain bonds. The bound IgG half-molecules, however, are not eluted from the support (such as antigen coated beads or Protein A immobilized on Sepharose gel) to which they are bound. Instead, the support is washed by suspending in a suitable buffer, followed by centrifugation, to remove the thiol reagent. The S-sulfonated half-molecules of the first IgG are then added to the bound sulfhydryl containing half-molecules of the second IgG in a suitably buffered medium, containing strontium chloride, and incubated at room temperature in an inert atmosphere. The hybrid antibodies so produced, formed on the support surface by nucleophilic displacement, can be eluted with a buffer containing an appropriate chaotropic agent (for example, high-salt concentration solution or surfactant).

The reaction products from each of the methods of selective cleavage and from the procedures for hybridization can be analyzed using the techniques described below.

Sodium dodecyl sulfate (SDS)-polyacrylamide gel electrophoresis (PAGE) under non-reducing conditions can be used to compare the electrophoretic mobilities of the unknown proteins or polypeptides to known proteins in order to estimate the molecular weights of the unknowns. This method permits the determination of the presence in reaction mixtures of individual heavy or light chains, half-molecules, or complete IgG molecules.

Isoelectric focusing in polyacrylamide gels can be used to separate proteins on the basis of differences in their isoelectric pH's, that is, the pH at which a protein has no net charge in a particular solvent. The presence of hybrid antibodies as different from either of the parent antibodies can be determined by this technique. This technique is more discriminating than SDS-PAGE.

Double diffusion in agar gel provides a method for detecting antigen binding by an antibody. Antigen and antibody placed in separate wells in the gel are allowed to diffuse toward each other. Binding of antigen by antibody results in a line of precipitate (insoluble antigen-antibody complexes) visible between the wells. Only bivalent antibodies will form precipitates with their antigens. Hybrid antibodies, which are monovalent with respect to each antigen, therefore, will not precipitate with either antigen.

Hybrid antibodies, prepared by the process of this invention, are useful in immunoassays requiring antibody recognition of two different antigens. In particular, they are useful in those circumstances in which the binding of a first antigen to the covalent hybrid antibody can modulate the binding of a second antigen to the hybrid antibody. The first antigen is an analyte of interest and the second antigen is an indicator such as an enzyme whose activity is modulated upon binding to the hybrid antibody.

By analyte of interest in the biological sample is meant the substance whose concentration is desired to be determined. The biological sample can be a biological fluid such as whole blood, blood serum, blood plasma, saliva, cerebral spinal fluid or urine or can be cell and tissue extracts. The analyte is often a protein present in one of these biological fluids but also includes drugs, hormones, vitamins, enzymes, antibodies, polysaccharides, bacteria, protozoa, fungi, viruses, cell and tissue antigens and other blood cell or blood fluid substances.

In the first aspect of the immunoassay of this invention, the indicator is an enzyme whose activity can be modulated upon binding to the hybrid antibody. A specific β-galactosidase is one

preferred enzyme for use in the immunoassay of this inventon. This enzyme is isolated from strain #W6101 *lac* $z^-$ of *E. coli* known as the AMEF (antibody modulated enzyme fragment) mutant. Antibodies to wild-type β-galactosidase can specifically activate this mutant enzyme. This modulation can also occur with univalent antibody fragments (Fab) and, therefore, by binding to one binding site on the hybrid antibody. Other potential indicator enzymes whose activities are modulated upon antibody binding include phenylalanine hydroxylase, penicillinase, glucose oxidase and human prostatic acid phosphatase. Other nonenzyme indicators can also be functional in the immunoassay method of this invention including fluorophores, radioactive materials, and bio- or chemiluminescent materials. While modulation will usually take the form of activation of indicator activity, inactivation is also possible.

The binding of analyte to the analyte specific binding site inhibits the binding of the indicator to the indicator-specific binding site of the hybrid antibody. Thus, since the indicator activity is not modulated when the analyte binding site is filled, the measured activity of the indicator, at the completion of the assay is proportional to the concentration of analyte in the biological fluid.

The immunoassay of this invention provides good sensitivity when compared to competitive-binding homogeneous immunoassays since the binding of one analyte molecule to the hybrid antibody substantially completely modulates the activity of the indicator, that is, it is a direct immunoassay. In conventional assays, more than one analyte molecule can be bound to the bivalent antibody and the modulation of the activity of the indicator or label may not be substantially complete upon the binding of a single analyte molecule to the antibody.

In certain circumstances, the analyte of interest is not sufficiently large to hinder the binding of the enzyme indicator to the covalent hybrid antibody. In these cases, an analyte conjugate (in known concentration) can be used in the method of this invention. The analyte conjugate and free analyte compete for binding to the hybrid antibody. The binding of the analyte conjugate sterically or ionically inhibits the binding of the enzyme indicator, thus preventing the modulation of activity. Such analyte conjugates, for example, can be analytes covalently bound to a high molecular weight carrier protein or to latex particles.

Optionally, the hybrid antibody can be modified, for example, by glutaraldehyde crosslinking to reduce the segmental flexibility in the hinge region, and thereby eliminating the need for a large analyte to sterically hinder the binding of the enzyme indicator.

The homogeneous hybrid antibody immunoassay of this invention can be performed by mixing and incubating the hybrid antibody, the enzyme indicator, and a biological fluid containing an unknown quantity of analyte. Reagents for the detection of the enzyme indicator can then be added to measure the modulated activity of the enzyme indicator. Optionally, an analyte conjugate can be utilized in the first mixing and incubation step for the measurement of analytes of low molecular weight. Also, optionally, the hybrid antibody can be pre-incubated with the biological fluid prior to adding the enzyme indicator.

The reagents for the detection of the enzyme indicator are dependent upon the enzyme indicator being utilized. These reagents can be chromogenic or fluorogenic substrates specific for the enzyme indicator. Other means of measuring enzyme activity such as electrochemically can also be employed in the method of this invention.

The measurement of color produced by the enzymatic cleavage of a chromogenic substrate is proportional to the modulated enzyme indicator activity. This activity, in turn, is a function of the amount of analyte in the biological fluid. Therefore, by performing the immunoassay on a series of samples containing known analyte concentrations, the unknown concentration of analyte in a biological fluid can be determined.

In a second aspect, the immunoassay of this invention also utilizes covalent hybrid antibody with one binding site specific for analyte and the other site specific for enzyme indicator. In this case, however, the activity of the enzyme indicator need not be modulated upon binding to the hybrid antibody. The hybrid antibody functions to bring the enzyme indicator and an enzyme-analyte conjugate in close spatial proximity. The enzyme indicator and a different second enzyme in the enzyme-analyte conjugate are chosen such that the product of one enzyme-catalyzed reaction is a substrate for the other enzyme. This way, when both enzymes are bound to the hybrid antibody in close spatial proximity (150Å), an enhancement of the rate of final product formation is observed. It should be noted that one of the two enzymes is not bound to the hybrid antibody directly but through the analyte with which it is conjugated. Free analyte from the biological fluid complete with the binding of the enzyme-analyte conjugate. Therefore, the higher the level of analyte, the less of the enzyme-analyte conjugate will be bound to the hybrid antibody leading to a lower rate enhancement. An inverse relationship is thus observed between the enzyme reaction rate and the concentration of analyte in the biological fluid.

A preferred enzyme system for use in this aspect of the invention is horseradish peroxidase as the indicator enzyme and glucose oxidase as part of the enzyme-analyte conjugate. Glucose oxidase catalyzes the production of hydrogen peroxide in the presence of glucose and water. The hydrogen peroxide thus formed is a substrate for horseradish peroxidase which, in the presence of a suitable chromogenic substrate, will catalyze color formation at a rate proportional to hydrogen peroxide concentration. As discussed above, the

rate of color formation is inversely related to the analyte concentration in the biological fluid.

In a third aspect, the immunoassay of this invention utilizes covalent hybrid antibody with one binding site specific for analyte and the other site specific for an aggregating substance, the indicator. By aggregating substance is meant a multivalent protein, a polyhapten-protein conjugate or hapten, each of which is attached to a latex particle. An example of latex particles useful in the method of this invention is described in EP—A1—73611.

The method of the third aspect of the invention can be carried out in the steps. The first step is a binding step in which the hybrid antibody, analyte from the biological fluid, and a polyanalyte (a signal generating reagent) such as a polyvalent analyte-protein conjugate, are mixed. The hybrid antibody can bind to either the free analyte from the biological fluid or to the polyanalyte with one of its binding sites. In the second step, the aggregating substance is added and the hybrid antibody-polyanalyte complexes combine with the aggregating substance to cause aggregation. This results in an increase in turbidity which can be measured spectrophotometrically. The level of aggregation is inversely proportional to the concentration of analyte in the biological fluid.

The process of this invention is also useful in the preparation of monovalent antibody fragments, i.e., half-molecules ($H_1L_1$). There are several reports in the literature of the utility of monovalent antibody fragments such as Fab and Fab' fragments. Fab fragments can be generated from intact IgG by limited proteolysis with the enzyme papain; they are monovalent with respect to antigen binding but lack the $F_c$ region of IgG. Fab' fragments can be generated by reduction of the inter-H chain disulfide bond(s) of $F(ab')_2$ fragments which, in turn, can be derived from pepsin cleavage of intact IgG. Fab' fragments are also monovalent but also lack the $F_c$ region. Half-molecules synthesized by the process of this invention are monovalent with respect to antigen binding, contain reactive sulfhydryl or thiosulfate group(s), and retain the important $F_c$ region. Their synthesis is rapid and requires no purification steps.

Example 1

Synthesis of a hybrid antibody

A. Preparation of S-sulfonated half-molecule

A commercially available IgG fraction from rabbit serum containing anti-horseradish peroxidase antibody (anti-HRP) was further purified by immunoaffinity chromatography on horseradish peroxidase-coupled agarose beads. The bound fraction containing anti-HRP was eluted from the affinity adsorbent with 2.5 M sodium thiocyanate in phosphate-buffered saline (PBS). The PBS buffer consisted of 0.14 M sodium chloride, 2 mM potassium chloride, 8 mM dibasic sodium phosphate, 1.5 mM dipotassium phosphate, and, optionally, 0.02% (w/v) sodium azide, at a pH of 7.4. The eluate was dialyzed against multiple changes of 0.1 M borate, pH 8.5, containing 4 mM ethylene diamine tetraacetic acid (EDTA). The final concentration of the anti-HRP was adjusted to 2 mg/mL, assuming a 1 mg/mL solution of IgG to have an optical density of 1.4 at 280 nm.

To 0.5 mL (1 mg) of this purified anti-HRP was added 6.26 mg solid sodium sulfite and 0.5 mg solid 5,5'-dithiobis(2-nitrobenzoic acid) (DTNB). The reaction mixture was stirred in a nitrogen atmosphere for 3 hours at room temperature and was then dialyzed overnight at room temperature against 20 mM N-tris(hydroxymethyl)methyl-2-aminoethanesulfonic acid (TES) buffer, pH 6.5, containing 1 mM EDTA. The product was S-sulfonated rabbit anti-HRP half-molecules.

Analysis under nonreducing conditions of the S-sulfonated rabbit anti-HRP on discontinuous SDS-polyacrylamide gels showed a single band of apparent molecular weight of 75,000 Daltons, corresponding to the half-molecule $H_1L_1$. Upon reduction with β-mercaptoethanol (βME, 5% v/v), two bands of apparent molecular weights of 55,000 and 23,000 Daltons were seen, corresponding to free heavy (H) chains and free light (L) chains, respectively.

B. Preparation of a reduced (SH) half-molecule

An Ig fraction was prepared from commercially available goat serum containing anti-glucose oxidase (anti-GO) by adding to the serum an equal volume of saturated ammonium sulfate, pH 7.0, in distilled water. This solution was stirred at room temperature for 30 minutes and was then centrifuged at 8000×g at 4°C. The supernatant liquid was decanted and the pellet was resuspended in saturated ammonium sulfate, centrifuged, and the supernatant liquid decanted again. The pellet was redissolved in half of the original serum volume of distilled water and the resultant solution dialyzed exhaustively against PBS. The dialyzed solution was then applied to protien A-coupled Sepharose CL-4B® gel (available from Pharmacia) in a sintered glass funnel. Approximately 5 mL of gel was used per 2.5 mL of Ig fraction. The gel was washed with 20 volumes of PBS and the bound fraction containing IgG was eluted with one volume of 0.1 M glycine, pH 3.0. The eluate was dialyzed exhaustively against PBS. The dialyzed solution was concentrated to a protein concentration of 2 mg/mL by ultrafiltration using an Amicon PM-30 membrane and its purity judged to be 95% by SDS-PAGE. This IgG fraction was then further purified by immunoaffinity chromatography on glucose-oxidase-coupled agarose beads. Elution of the anti-GO was performed as described above for anti-HRP. The final concentration was adjusted to 2 mg/mL.

To 1.0 mL (2 mg) of this purified goat anti-GO was added 1 mL of GO-coupled Sepharose 4B®, prepared from CNBr-activated Sepharose 4B. The approximate ratio of GO to Sepharose was 2 mg/mL of wet gel. The antibody was incubated

with the gel for one hour at room temperature with constant mixing. After this time, the gel was centrifuged in a clinical centrifuge and the supernatant withdrawn. The optical density of the supernatant was determined at 280 nm. Using a value of 1.4 for the optical density of 1 mg/mL solution of IgG, it was estimated that approximately 50% of the anti-GO antibody was bound to the gel. The gel was washed three times by suspension in 0.1 M borate/4 mM EDTA, pH 8.5, followed by centrifugation and aspiration of the supernatant. After the final wash, the pellet was resuspended in an equal volume of 14 mM βME in 0.1 M borate/4 mM EDTA, pH 8.5, and incubated with shaking for 30 minutes at room temperature under a blanket of nitrogen. The gel was separated from suspension by centrifugation, the supernatant aspirated, and the gel eluted with 0.1 M glycine/4 mM EDTA, pH 3.0. The eluate was dialyzed overnight against 0.02 M glycine/4 mM EDTA, pH 3.0, through which nitrogen was bubbled continuously. The product was reduced (SH) goat anti-GO.

Analysis by SDS-PAGE of the reduced goat anti-GO preparation under nonreducing conditions revealed a single major band of apparent molecular weight of 75,000 Daltons. Two minor bands (accounting for less than 10% by weight of the total protein) also appeared having apparent molecular weights of 55,000 and 23,000 Daltons, respectively. These three correspond, respectively, to half-molecules ($H_1L_1$), free H chains, and free L chains. Upon addition of βME (5% v/v), the 75,000 Dalton band was converted to the 55,000 and 23,000 Dalton bands, as expected.

C. Preparation of hybrid antibody

Equal amounts of the S-sulfonated rabbit anti-HRP and the reduced goat anti-GO were mixed and transferred to dialysis tubing. The resultant solution was dialyzed for 24 hours at room temperature against 20 mM TES, 1 mM EDTA, and 0.1 M strontium chloride, pH 6.5, through which nitrogen was bubbled continuously. The mixture was then dialyzed against PBS for 24 hours.

Analysis of the dialyzed solution by SDS-PAGE under nonreducing conditions revealed a single major band of apparent molecular weight of 150,000 Daltons which, upon reduction (5% βME, v/v), yielded two bands of apparent molecular weights 55,000 and 23,000 Daltons, respectively. These three molecular weights correspond to the product covalent hybrid antibody, free H chains, and free L chains. (Less intense bands at 55,000 and 23,000 Daltons were detected under nonreducing conditions when the gels were overloaded. These bands probably represent the free H and free L chains generated during the reduction of anti-GO.)

The covalent hybrid antibody was further characterized by double diffusion in agar gel: when the parent antibodies, anti-HRP and anti-GO, were diffused against HRP and GO, respectively, precipitin bands were observed, while the hybrid antibody formed by the process of this invention from S-sulfonated anti-HRP and reduced anti-GO failed to form a precipitate with either HRP or GO. Also, when S-sulfonated anti-HRP was diffused against (HRP or when reduced (SH) anti-GO was diffused against GO, no precipitin lines were formed confirming the monovalent nature of these half-molecules with respect to antigen binding. Precipitin lines were seen, however, when the hybrid antibody was diffused against either sheep anti-rabbit IgG or sheep anti-goat IgG, indicating the dual (hybrid) character of the antibody.

The hybrid antibody prepared above was still further analyzed by solid phase immunoprecipitation for its ability to bind an enzyme antigen. Twenty-five microliters of hybrid antibody was incubated for 1 hour at room temperature with 10 µl of glucose oxidase (1 mg/mL in PBS). 75 µl of a suspension of goat anti-rabbit IgG-coated latex beads (available from Bio-Rad) in PBS containing 1% ovalbumin (OVA) and 0.05% Triton X-100® (a nonionic surfactant available from Rohm and Raas Company) was added to the above and incubated, with shaking, for 1 hour at room temperature. The suspension was centrifuged and the pellet washed in the PBS/OVA/Triton® X-100 buffer three times by centrifugation. After the final wash, the beads were resuspended in 500 µl of 0.1 M glucose in PBS, 100 µl of o-phenylenediamine (OPD) (5 mM), and 10 µl of horseradish peroxidase (1 mg/mL in PBS). The formation of a color was monitored spectrophotometrically at 460 nm. Controls for this assay included the use of self-hybridized goat anti-glucose oxidase in place of the rabbit/goat hybrid antibody, and assays using either no antibody or no glucose oxidase.

The basis of color formation in this assay was as follows: the hybrid antibody was bound to goat anti-rabbit IgG-coated beads by virtue of its rabbit (HRP binding) character. Glucose oxidase was precipitated by these beads only if hybrid antibody was present. (Presence of intact goat anti-glucose oxidase or of half-molecules of the same will not affect the assay, since these will not be precipitated by the goat anti-rabbit IgG-coated beads. This was verified by a control assay utilizing goat anti-glucose oxidase which has been hybridized with itself in a manner similar to that described in this Example.) After washing to remove any free glucose oxidase, glucose, OPD, and HRP were added. Glucose oxidase hydrolyzed glucose to yield $H_2O_2$, which served as the substrate for HRP. Color was generated only in the presence of glucose oxidase bound by the hybrid antibody (all free GO having been washed out).

When assayed as described, the hybrid antibody synthesized in this Example led to color formation. Elimination of the antibody or the glucose oxidase from the assay resulted in no color formation. When half-hybridized goat anti-glucose oxidase was substituted for the rabbit/goat hybrid, no color resulted. These results

proved that the desired hybrid antibody product was obtained.

Example 2

Preparation of IgG half-molecules through reduction with β-mercaptoethanol of immobilized antibodies

Protein A-Sepharose CL-4B gel was swollen in 0.1 M borate/4 mM EDTA buffer, pH 8.5. To 50 µl of a 50% (v/v) suspension was added 100 µl of a 1 mg/mL solution of rabbit anti-horseradish peroxidase IgG. This was incubated for 1 hour at room temperature with constant agitation and then centrifuged. The supernatant liquid was aspirated and the pellet washed three times with 0.1 M borate/4 mM EDTA buffer, pH 8.5. After the last wash, the pellet was resuspended in 100 µl of 14 mM β-mercaptoethanol in the same buffer and incubated under nitrogen for 15 minutes at room temperature. It was then centrifuged, the supernatant liquid aspirated, and the pellet washed three times with the borate/EDTA buffer. The pellet contained reduced half-molecules bound to the Protein A-Sepharose gel. The yield of the reduced rabbit anti-HRP half-molecules was 75%, 90% of which remained bound to the gel through the washing steps. The bound half-molecules can be utilized as is (see Example 3) or could be eluted from the gel by 0.1 M glycine/1 mM EDTA buffer, pH 3.0. The product was characterized utilizing the procedures as described above.

Example 3

Preparation of hybrid antibody

An equivalent weight of S-sulfonated goat anti-GO half-molecules, prepared as shown in Example 1, Part A, in 20 mM TES/1 mM EDTA/0.1 M $SrCl_2$ was added to the pellet prepared in Example 2 above. The pellet was resuspended and agitated overnight at room temperature under nitrogen. The supernatant was then removed and the pellet was washed once with 20 mM TES/l mM EDTA/0.1 M $SrCl_2$ and twice more with TES/EDTA. The bound product IgG hybrid antibody was eluted with 0.1 M glycine, pH 3.0, and dialyzed into PBS. The product was characterized utilizing the procedures described above.

Example 4

Preparation of IgG half-molecules through reduction with β-mercaptoethylamine

To 900 µl of rabbit IgG (2 mg/mL) was added 100 µl of 1 M β-mercaptoethylamine in 20 mM TES/1 mM EDTA, pH 6.5. Incubation was carried out for 2 hours at 37°C, after which the sample was passed over Sephadex G25® gel equilibrated in TES/EDTA. Approximately 80% of the IgG was converted to reduced half-molecules under these conditions. The remaining 20% was H-chain dimers and free L chains. The reduced half-molecules so produced can be utilized to produce covalent hybrid antibodies in accordance with Example 1, Part C. The product was characterized utilizing the procedures described above.

Example 5

Alternative synthesis of covalent hybrid antibody

Goat anti-glucose oxidase, purified as described in Example 1, Part B, can be S-sulfonated as described for the rabbit anti-HRP in Example 1, Part A. After the dialysis step to remove excess DTNB, the reaction mixture can be adjusted to a concentration of 0.014 M in βME and dialyzed overnight against a buffer containing 20 mM glycine, 4 mM EDTA, 0.1 M $SrCl_2$, at a pH of 3.0. The solution can then be hydridized with the S-fulfonated rabbit anti-HRP, prepared as in Example 1, Part A, as described in Example 1, Part C.

The yield of the desired hybrid antibody is such a synthesis is expected to approach 90%, since the formation of the reduced anti-glucose oxidase by this S-sulfonation route has a yield of approximatley 90%.

Example 6

Synthesis of half-molecules from monoclonal antibodies

S-sulfonated half-molecules were prepared and characterized as described in Example 1, Part A, using monoclonal antibodies. The yield of the desired S-sulfonated half-molecule was 100%. The monoclonal antibodies were anti-theophilline (heavy chain subclass $\gamma_1$ and light chain isotype κ) and anti-digoxin (heavy chain subclass $\gamma_3$ and light chain isotype κ). The starting material monoclonal antibodies were affinity purified from ascites fluid, concentrated by ultrafiltration to a concentration of 2 mg/mL, and typed as to light chain isotype and heavy chain subclass by double diffusion in agar gel.

Example 7

Enzyme activation immunoassay employing a hybrid antibody reagent

An immunoassay for a large molecular weight analyte will utilize the following materials:

(a) covalent hybrid antibody reagent as described in Example 1 Part C above, having binding specificities for the analyte and an enzyme indicator;

(b) signal-producing enzyme indicator reagent, whose activity is modulated by binding to the covalent hybrid antibody;

(c) reagents for the colorimetric or fluorimetric signal-generating reaction; and

(d) biological sample containing analyte which is a protein of molecular weight ≥10,000 but more often ≥100,000.

The biological sample can be a serum sample, a urine sample or a whole blood sample. The enzyme indicator can be an enzyme or enzyme subunit which is inactive unless bound by antibody; for example, the AMEF mutant of *E. coli* β-galactosidase or human prostatic acid phosphatase. The enzyme indicator can also be an active enzyme which is inactivated by the binding of antibody. The reagents for the signal-generating reaction are chromogenic or fluorogenic substrates of the enzyme indicator plus any additional necessary cofactors or cations in an aqueous buffer of pH 4—10.

It is expected that materials (a), (b), and (d) will be incubated together for a time at a temperature between 0° and 50°C, more often between 22° and 46°C, before adding the reagents (c). However, in certain instances, it may be desirable to preincubate component (a) and (d) before the addition of the enzyme indicator. Likewise, it is possible that all materials are added simultaneously. The length of incubation will usually be less than 30 minutes, more often 1—4 minutes. Measurement of the signal produced by the enzyme will usually be made in less than 30 minutes after addition of material (c) and more often in less than 3 minutes.

Where the enzyme indicator is inactive and becomes active only when bound by antibody, the amount of signal produced will be inversely proportional to the concentration of analyte in the sample. Where the enzyme-indicator is active and is inactivated when bound by antibody, the amount of signal produced will be directly proportional to the concentration of analyte in the sample.

## Claims

1. A substantially pure immunoglobulin heavy chain-light chain half-molecule having the structure R—S—$SO_3$—, where R is $H_1L_1$.

2. An immunoglobulin half-molecule according to claim 1 which has antigen-binding specificity for digoxin and is obtained from monoclonal antibody to digoxin.

3. An immunoglobulin half-molecule according to claim 1 which has antigen-binding specificity for theophylline and is obtained from monoclonal antibody to theophylline.

4. A process for preparing S-sulfonated immunogloblin half-molecules by selectively cleaving an immunoglobulin molecule into its heavy chain-light chain half-molecules by sulfitolysis of the inter-heavy chain disulfide linkage with sodium sulfite in the presence of 5,5'-dithiobis(2-nitrobenzoic acid).

5. A process for preparing reduced immunoglobulin half-molecules by selectively cleaving an immunoglobulin molecule into its heavy chain-light chain half-molecules by sulfitolysis of the inter-heavy chain disulfide linkage with sodium sulfite in the presence of 5,5'-dithiobis(2-nitrobenzoic acid) followed by reduction of the S-sulfonated half-molecules.

6. A process for preparing reduced immunoglobulin half-molecules by selectively cleaving an immunoglobulin molecule bound to an immobilized receptor into its heavy chain-light chain half-molecules by reduction of the inter-heavy chain disulfide linkage.

7. A substantially pure covalent hybrid antibody consisting essentially of two different heavy chain-light chain half-molecules, wherein the first of said half-molecules provides a binding site for a first antigen and the second of said half-molecules provides a chemically different binding site for the first antigen or a second antigen; and wherein said half-molecules are bonded to each other through disulfide linkage.

8. The covalent hybrid antibody of Claim 7 wherein the first and second half-molecules provide chemically different binding sites for different antigens.

9. The covalent hybrid antibody of Claim 8 wherein the first half-molecule provides a binding site for an analyte and the second half-molecule provides a binding site for an indicator.

10. A process for preparing covalent hybrid antibodies comprising the steps of:

(A) selectively cleaving a first immunoglobulin molecule which is an antibody to a first antigen into its heavy chain-light chain half-molecules by sulfitolysis of the inter-heavy chain disulfide linkage with sodium sulfite in the presence of 5,5'-dithiobis(2-nitrobenzoic acid to produce S-sulfonated half-molecules;

(B) selectively cleaving a second immunoglobulin molecule which is an antibody to the first or a second antigen into its heavy chain-light chain half-molecules by reduction of the inter-heavy chain disulfide linkage to produce reduced half-molecules; and

(C) combining the S-sulfonated half-molecules from step (A) with the reduced half-molecules from step (B).

11. A homogeneous immunoassay for the measurement of analyte comprising the steps of incubating sequentially or simultaneously:

(A) a substantially pure covalent hybrid antibody consisting essentially of two different heavy chain-light chain half-molecules, wherein the first of said half-molecules provides a binding site for a first antigen and the second of said half-molecules provides a chemically different binding site for the first or a second antigen; wherein said half-molecules are bonded to each other through disulfide linkage;

(B) a biological sample containing analyte;

(C) an indicator; and

(D) reagents for signal-generating reaction.

12. The homogeneous immunoassay of Claim 11 wherein the indicator is an enzyme and the reagents for signal-generating reaction include enzyme substrates.

13. The homogeneous immunoassay of Claim 11 wherein the indicator is an aggregating substance and the reagents for signal-generating reaction include polyanalytes.

## Patentansprüche

1. 'Im wesentlichen reine Immunoglobulin-schwere-Ketteleichte-Kette-Halbmoleküle mit der Struktur R—S—$SO_3$—, worin R $H_1L_1$ ist.

2. Immunoglobulin-Halbmolekül nach Anspruch 1, das Antigen-Bindungsspezifität für Digoxin aufweist und aus monoklonalen Antikörpern auf Digoxin erhalten wird.

3. Immunoglobulin-Halbmolekül nach Anspruch 1, das Antigen-Bindungsspezifität für

Theophyllin aufweist und aus monoklonalen Antikörpern auf Theophyllin erhalten wird.

4. Verfahren zur Herstellung von S-sulfonierten Immunoglobulin-Halbmolekülen durch selektives Spalten eines Immunoglobulinmoleküls in seine Schwere-Kette-leichte-Kette-Halbmoleküle durch Sulfitolyse der Interschwere- Kette-Disulfidbindung mit Natriumsulfit in Gegenwart von 5,5'-Dithiobis(2-nitrobenzoesäure).

5. Verfahren zur Herstellung reduzierter Immunoglobulin-Halbmoleküle durch selektives Spalten eines Immunoglobulinmoleküls in seine Schwerte-Kette-leichte-Kette-Halbmoleküle durch Sulfitolyse der Inter-schwere-Kette-Disulfid-Bindung mit Natriumsulfit in Gegenwart von 5,5'-Dithiobis(2-nitrobenzoesäure), gefolgt durch Reduktion der S-sulfonierten Halbmoleküle.

6. Verfahren zur Herstellung reduzierter Immunoglobulin-Halbmoleküle durch selektive Spaltung eines an einen immobilisierten Rezeptor gebundenen Immunoglobulin-Moleküls in seine Schwere-Kette-leichte-Kette-Halbmoleküle durch Reduktion der Inter-schwere-Kette-disulfidbindung.

7. Im wesentlichen reiner, kovalenter, hybrider Antikörper, im wesentlichen bestehend aus zwei verschiedenen Schwere-Kette-leichte-Kette-Halbmolekülen, worin das erste dieser Halbmoleküle eine Bindungsstelle für ein erstes Antigen bereitstellt und das zweite dieser Halbmoleküle eine chemisch verschiedene Bindungsstelle für das erste Antigen oder eine zweites Antigen bereitstellt; und worin diese Halbmoleküle durch Disulfidbindung aneinander gebunden sind.

8. Kovalenter, hybrider Antikörper nach Anspruch 7, worin das erste und das zweite Halbmolekül chemisch verschiedene Bindungsstellen für verschiedene Antigene bereitstellen.

9. Kovalenter, hybrider Antikörper nach Anspruch 8, worin das erste Halbmolekül eine Bindungsstelle für ein Analysat bereitstellt und das zweite Halbmolekül eine Bindungsstelle für einen Indikator bereitstellt.

10. Verfahren zur Herstellung kovalenter, hybrider Antikörper, welches die Schritte umfasst:

(A) selektives Spalten eines ersten Immunoglobulinmoleküls, das ein Antikörpers auf ein erstes Antigen ist, in seine Schwere-Kette-leichte Kette-Halbmoleküle durch Sulfitolyse der Inter-schwere-Kette-Disulfidbindung mit Natriumsulfit in Gegenwart von 5,5'-Dithiobis(2-nitrobenzosäure) unter Bildung S-sulfonierter Halbmoleküle;

(B) selektives Spalten eines zweiten Immunoglobulinmoleküls, das ein Antikörper auf das erste oder ein zweites Antigen ist, in seine Schwere-Kette-leichte-Kette-Halbmoleküle durch Reduktion der Inter-schwere-Kette-Disulfidbindung unter Bildung reduzierter Halbmoleküle; und

(C) Vereinigen der S-sulfonierten Halbmoleküle aus Schritt (A) mit den reduzierten Halbmolekülen aus Schritt (B).

11. Homogener Immunoassay zur Messung eines Analysats durch aufeinanderfolgendes oder gleichzeitiges Inkubieren:

(A) eines im wesentlichen reinen, kovalenten, hybriden Antikörpers, im wesentlichen bestehend aus zwei verschiedenen Schwere-Kette-leichte-Kette-Halbmolekülen, worin das erste dieser Halbmoleküle eine Bindungsstelle für ein erstes Antigen bereitstellt und das zweite dieser Halbmoleküle eine chemisch verschiedene Bindungsstelle für das erste oder ein zweites Antigen bereitstellt; worin diese Halbmoleküle durch Disulfidbindungen aneinandergebunden sind;

(B) einer das Analysat enthaltenden biologischen Probe;

(C) eines Indikators; und

(D) von Reagentien für eine Signal-erzeugende Reaktion.

12. Homogener Immunoassay nach Anspruch 11, worin der Indikator ein Enzym ist und die Reagentien für die Signal-erzeugende Reaktion Enzymsubstrate einschliessen.

13. Homogener Immunoassay nach Anspruch 11, worin der Indikator eine aggregierende Substanz ist und die Reagentien für die Signal-erzeugende Reaktion Polyanalysate einschliessen.

**Revendications**

1. Une demi-molécule à chaîne lourde-chaîne légère d'immunoglobuline sensiblement pure ayant la structure R—S—$SO_3$—, où R est $H_1L_1$.

2. Une demi-molécule d'immunoglobuline selon la revendication 1, qui a une spécificité de fixation d'antigène pour la digoxine et est obtenue à partir d'anticorps monoclonal à la digoxine.

3. Une demi-molécule d'immunoglobuline selon la revendication 1, qui a une spécificité de fixation d'antigène pour la théophylline et est obtenue à partir d'anticorps monoclonal à la théophylline.

4. Un procédé pour la préparation de demi-molécules d'immunoglobulines S-sulfonées en clivant sélectivement une molécule d'immunoglobuline en ses demi-molécules à chaîne lourde-chaîne légère par sulfitolyse de la liaison disulfure entre chaînes lourdes avec du sulfite de sodium en présence de 5,5'-dithiobis(acide 2-nitrobenzoïque).

5. Un procédé pour la préparation de demi-molécules d'immunoglobulines réduites en clivant sélectivement une molécule d'immunoglobuline en ses demi-molécules à chaîne lourde-chaîne légère par sulfitolyse de la liaison disulfure entre chaînes lourdes avec du sulfite de sodium en présence de 5,5'-dithiobis(acide 2-nitrobenzoïque) suivie d'un réduction des demi-molécules S-sulfonées.

6. Un procédé pour la préparation de demi-molécules d'immunoglobulines réduites en clivant sélectivement une molécule d'immunoglobuline liée à un récepteur immobilisé en ses demi-molécules à chaîne lourde-chaîne légère par

réduction de la liaison disulfure entre chaînes lourdes.

7. Un anticorps hybride covalent sensiblement pur constitué essentiellement de deux demi-molécules à chaîne lourde-chaîne legère différentes, où la première des demi-molécules fournit un site de fixation pour un premier antigène et la seconde des demi-molécules fournit un site de fixation chimiquement différent pour le premier antigène ou un second antigène; et où les demi-molécules sont liées l'une à l'autre par une liaison disulfure.

8. Un anticorps hybride covalent selon la revendication 7, dans lequel la première et le seconde demi-molécules fournissent des sites de fixation chimiquement différents pour des antigènes différents.

9. Un anticorps hybride covalent selon la revendication 8, dans lequel la première demi-molécule fournit un site de fixation pour un analyte et la seconde demi-molécule fournit un site de fixation pour un indicateur.

10. Un procédé pour la préparation d'anticorps hybrides covalents comprenant les étapes qui consistent à:

(A) cliver sélectivement une première molécule d'immunoglobuline qui est un anticorps pour un premier antigène en ses demi-molécules à chaîne légère-chaîne lourde par sulfitolyse de la liaison disulfure entre chaînes lourdes avec du sulfite de sodium en présence de S,S'-dithiobis(acide 2-nitrobenzoïque) de façon à produire des demi-molécules S-sulfonées;

(B) cliver sélectivement une seconde molécule d'immunoglobuline qui est un anticorps pour le premier ou un second antigène en ses demi-molécules à chaîne lourdechaîne légère par réduction de la liaison disulfure entre chaînes lourdes de façon à produire des demi-molécules réduites; et

(C) combiner les demi-molécules S-sulfonées résultant de l'étape (A) avec les demi-molécules réduites de l'étape (B).

11. Une détermination immunologique homogène pour la mesure d'un analyte, comprenant les étapes qui consistent à faire incuber successivement ou simultanément:

(A) un anticorps hybride covalent sensiblement pur constitué essentiellement de deux demi-molécules à chaîne lourde-chaîne légère différentes, où la première des demi-molécules fournit un site de fixation pour un premier antigène et la seconde des demi-molécules fournit un site de fixation chimiquement différent pour le premier ou un second antigène; et où les demi-molécules sont liées l'une à l'autre par une liaison disulfure;

(B) un échantillon biologique contenant un analyte;

(C) un indicateur; et

(D) des réactifs pour une réaction produisant un signal.

12. Une détermination immunologique homogène selon la revendication 11, dans laquelle l'indicateur est un enzyme et les réactifs pour une réaction produisant un signal comprennent des substrate pour enzymes.

13. Une détermination immunologique homogène selon la revendication 11, dans laquelle l'indicateur est une substance agglutinante et les réactifs pour une réaction produisant un signal incluent des polyanalytes.